# EUROPEAN PATENT APPLICATION

(11) **EP 2 174 626 A1**
(43) Date of publication of application: **14.04.2010**
(21) Application number: 09172093.8
(22) Date of filing: 02.10.2009
(51) Int. Cl.: A61F 7/03, A61F 7/02

(54) **Exothermic structure that is directly applied to skin and method for preparing the structure**

(30) Priority: 07.10.2008 JP 2008260260
(71) Applicant: Ohshin MLP Co., Ltd, Echizen-shi Fukui 915-0052 (JP)
(72) Inventor: Ota, Keizo, Fukui 915-0052 (JP); Ota, Michiko, Fukui 915-0052 (JP); Hata, Satoko, Fukui 915-0052 (JP)
(74) Representative: Giovannini, Francesca

(57) **Abstract**

An exothermic structure that is directly applied to skin comprises a pouch having an air-permeable side and an air-impermeable side, and a exothermic composition that is incorporated into the pouch in a sealed state. The air-impermeable side comprises an inner polymer sheet layer, an intermediate adhesive, and an outer release sheet, wherein the adhesive layer exists in part or entire of an outer periphery on an inner surface of the release sheet. The total area of the adhesive layer is 10 to 40% of the area of the release sheet, the adhesive layer has an adhesive portion(s) and a non-adhesive portion(s) or only an adhesive portion(s), the total area of the adhesive portion(s) is 30 to 100% of the total area of the adhesive layer, and in the outer periphery the total length of the adhesive portion(s) is 60 to 100% of the total length of the adhesive layer.

## Description

### BACKGROUND OF INVENTION

### 1. Field of the Invention

This invention relates to an exothermic structure that is used by directly applying to skin and method for preparing the structure.

### 2. Background Art

An exothermic structure of a disposable type, so-called a disposable body warmer, has been known as a device for simply warming up a part of human body. Among those exothermic structures, some ones are used by directly sticking on underwear or by directly applying to skin. Especially for the latter one, two requirements should be satisfied, namely, a prevention of the exothermic structure from peeling off during its use and another prevention of an occurrence of dermatitis such as a rubor, a rash, and the like even after a long-term use.

To satisfy these requirements, U. S. patent publication No. 2006/0282138 Al discloses an exothermic structure that is directly applied to skin comprising an adhesive layer that is partly made, wherein the adhesive layer has a specific adhesive force. The adhesive layer of the exothermic structure has a shape of, for example, check, and has portions where there is no tackiness agent. The adhesive layer having the shape of check exists entirely on one side of the exothermic structure.

Japanese Patent Early-publication No. Hei. 11-209272 discloses a patch. In the patch, medicine-holding pieces are held inside on an air-permeable support comprising adhesive layer portions, wherein many adhesive layer portions are sprinkled at intervals.

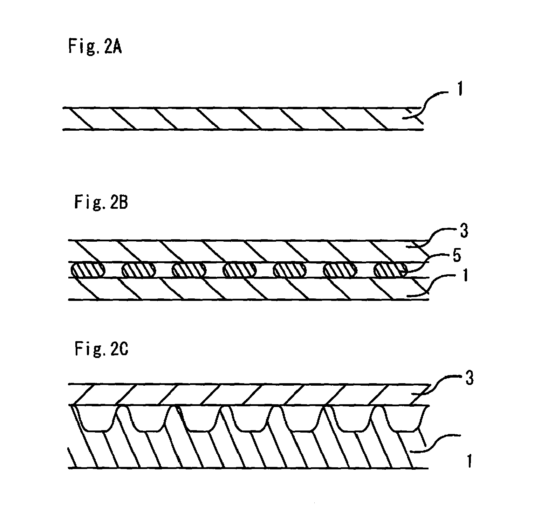

Japanese Patent Early-publication No. Hei. 7-61414 discloses an apparatus for producing a disposable body warmer. Japanese Patent Early-publication No. 2003-205556 discloses a method for producing two exothermic structures at the same time in paragraph number 0016 and figure 2.

### SUMMARY OF INVENTION

The inventors of this invention have thought that to reduce more and more the risk of low-temperature burn when the exothermic structure is used, the degree of adhesion of the portion where there is an exothermic composition to skin should be reduced more and more. Thus, to provide an exothermic structure that satisfies the above requirement and at the same time another requirement that the exothermic structure is not peeled off during its use, they have studied.

Further, they have also studied to develop a new method for preparing exothermic structure. In the method, a pouch into which an exothermic composition is put in a sealed state is made by heat-sealing, an adhesive layer having no or mild irritation to skin can be used, and to increase production efficiency two or more exothermic structures that stand in line can be produced at once in an excellent dimensional accuracy.

This invention intends to provide an exothermic structure that is directly applied to skin, wherein an adhesive layer exists only in an outer periphery of the exothermic structure and has a necessary adhesive force.

Further, this invention intends to provide a method for preparing efficiently the exothermic structure that is directly applied to skin.

The inventors of this invention have extremely studied to attain the above objects. As a result, they have accomplished this invention.

Namely, this invention relates to an exothermic structure that is directly applied to skin comprising a pouch having an air-permeable side and an air-impermeable side, and a exothermic composition that is incorporated into the pouch in a sealed state, wherein the air-impermeable side comprises an inner polymer sheet layer, an intermediate adhesive layer that is used to apply to skin, and an outer release sheet, wherein the adhesive layer exists in part or entire of an outer periphery on an inner surface of the release sheet, the total area of the adhesive layer is from 10 to 40% of the area of the release sheet, the adhesive layer has an adhesive portion(s) and a non-adhesive portion(s) or only an adhesive portion(s), the total area of the adhesive portion(s) is from 30 to 100% of the total area of the adhesive layer, and in the outer periphery the total length of the adhesive portion(s) is from 60 to 100% of the total length of the adhesive layer.

The air-impermeable side comprises, as essential constituent features, an inner polymer sheet layer, an outer release sheet, and an adhesive layer that is used to apply to skin, wherein the adhesive layer exists in part or entire of the outer periphery on the inner surface of the release sheet. The air-impermeable side may comprise an inner polymer sheet layer, a layer of a tackiness agent that exists on an outer surface of the polymer sheet layer, a fibrous layer that is bonded onto the layer of the tackiness agent, a release sheet, and an adhesive layer that is used to apply to skin, wherein the adhesive layer exists between the fibrous layer and the release sheet.

It is preferable that the adhesive layer that is directly applied to skin exists or is formed on an inner surface of the release sheet only in part or entire of a portion that corresponds to a portion where the air-permeable side and the air-impermeable side are bonded together.

The term "part" means that (1) the adhesive layer comprises an adhesive portion(s) and an non-adhesive portion(s) or that (2) in an outer periphery or a portion that corresponds to a portion where the air-permeable side and the air-impermeable side are bonded together, the adhesive layer consisting of only the adhesive portion is clearly discontinuous.

The exothermic structure that is directly applied to skin comprises those that have the following features:
(1) an inner periphery of the adhesive layer exists at a position that is outside of an inner periphery of a portion that corresponds to a portion where the air-permeable side and the air-impermeable side are bonded together;
(2) the distance between the outer periphery and the inner periphery of the adhesive layer is from 5 to 30 mm;
(3) the adhesive layer has the non-adhesive portion(s);
(4) the shape of the non-adhesive portion(s) is diamond; and
(5) the adhesive layer has two U-like-shaped portions that are axisymmetric to each other, and the distance between the U-like-shaped portions is from 10 to 30 mm.

The exothermic structure that is directly applied to skin is not restricted to one having only one pouch into which an exothermic composition is put. This invention also comprises an exothermic structure having two or more pouches into which an exothermic composition is put.

This invention also relates to a process for preparing, among the above exothermic structures, one having an air-impermeable side which comprises an inner polymer sheet layer, a layer of a tackiness agent that exists on an outer surface of the polymer sheet layer, a fibrous layer that is bonded onto the layer of the tackiness agent, an outer release sheet, and an adhesive layer that is used to apply to skin, wherein the adhesive layer exists between the fibrous layer and the release sheet. This process comprises (1) setting a first sheet that is to be an air-permeable side comprising an outer-most layer a that is at least one of the outer-most layers of the first sheet and a second sheet comprising a polymer sheet b and a layer of a tackiness agent that exists on the entire of one surface of the polymer sheet b and of which surface is protected by a release paper so that the outer-most layer a of the first sheet faces the polymer sheet b of the second sheet, wherein the outer-most layer a is a heat-sealable by itself or can be heat-sealed by the polymer sheet b of the second sheet, and the polymer sheet b is a heat-sealable by itself or can be heat-sealed by the outer-most layer a of the first sheet, (2) heat-sealing in a transverse direction (with the proviso that if a heat-sealed portion in the transverse direction has been previously made, this step is not conducted), (3) heat-sealing in a longitudinal direction to form parallel at least two pouch portions each having heat-sealed three sides, (4) putting an exothermic composition into those pouch portions, (5) heat-sealing in a transverse direction to make pouch portions into which the exothermic composition is put in a sealed state, (6) removing the release paper, (7) applying a sheet for a fibrous layer to the layer of the tackiness agent that has been bared, wherein the sheet comprises the fibrous layer, on portions that correspond to the outer peripheries of pouches that would be made and on one surface of the fibrous layer, an adhesive layer that is directly applied to skin, and a release sheet that covers the adhesive layer, so that a side of the fibrous layer on which side there is no adhesive layer faces the layer of the tackiness agent, (8) cutting, at need, the heat-sealed portion(s) of the longitudinal direction at a center of the width of the longitudinal heat-sealed portion(s), (9) simultaneously or posteriorly cutting the transverse heat-sealed portion at a center of the width of the transverse heat-sealed portion to give an exothermic structure(s), and then (10) putting the exothermic structure(s) into an air-proof outer bag(s) in a sealed state.

The term "transverse direction" means a vertical direction against the traveling direction of the production line. The term "longitudinal direction" means a direction that is the same as the traveling direction of the production line.

In the above process, the step (2) means the following situation. When the production starts, to form the bottoms of pouch portions the heat-sealing of the traveling direction must be done. However, after the pouch portions were made into which an exothermic composition had been put in a sealed state, the bottoms of the pouch portions have been formed. This is because to close an opening of a pouch portion (namely, a portion that has not been heat-sealed), wherein the opening was used to put the exothermic composition into the pouch portion, a transverse heat-sealing is done and then the a transverse heat-sealed portion is cut at a center of the width of the transverse heat-sealed portion. Thus, after the pouch portions of a first line in a longitudinal direction was made, it is unnecessary to do the transverse heat-sealing of the first sheet with the second sheet.

The step (8) may be done at need. For example, in the case where by the production line two pouch portions that form a transverse line (namely, two pouch portions that line a vertical direction against the traveling direction of the production line) can be simultaneously made, and an exothermic structure comprising two pouch portions that form a transverse line is to be produced, the step (8) is not conducted. However, in the case where by the above production line an exothermic structure comprising only one pouch portion is to be produced, cut of the heat-sealed portion of the longitudinal direction that exists between the two pouch portions at a center of the width of the longitudinal heat-sealed portion is done. For example, in the case where by the production line four pouch portions that form a transverse line (namely, four pouch portions that line a vertical direction against the traveling direction of the production line) can be simultaneously made, and an exothermic structure comprising two pouch portions that form a transverse line is to be produced, cut is done at only the longitudinal heat-sealed portion that exists at a center among five longitudinal heat-sealed portions. If an exothermic structure comprising only one pouch portion is to be produced by the same production line, cuts are done at the three longitudinal heat-sealed portions each existing between two pouch portions.

The cut of the transverse heat-sealed portion at a center of the width of the transverse heat-sealed portion means not only the case where cuts are done in all transverse heat-sealed portions but also the case where cuts are done at parts of transverse heat-sealed portions. For example, an exothermic structure comprising two pouch portions that line the longitudinal direction (namely, the traveling direction of the production line) is to be produced, the cut of the transverse heat-sealed portion is alternately done.

The cut of the heat-sealed portion of the longitudinal direction at the center of the width of the longitudinal heat-sealed portion may be preferably done by using a rotary blade. The cut of the heat-sealed portion of the transverse direction at the center of the width of the transverse heat-sealed portion may be preferably done by using a roll cutter.

### BRIEF DESCRIPTION OF DRAWINGS

Figure 1 is a schematic, sectional view of one example of the exothermic structure that is directly applied to skin according to this invention.
Figures 2A, 2B, and 2C are schematic, sectional views of examples of the air-permeable side.
Figure 3 is a schematic, plane view of one example of the air-permeable side.
Figure 4 is a schematic, sectional view of one example of the air-impermeable side.
Figure 5 is a schematic, sectional view of another example of the air-impermeable side.
Figures 6A and 6B are schematic, plane views of examples of the adhesive layers that are used to apply to skin, which show the sizes and shapes of the adhesive layers.
Figures 7A and 7B are schematic, plane views of examples of the adhesive layers that are used to apply to skin, which show the sizes and shapes of the adhesive layers.
Figures 8A and 8B are schematic, plane views of examples of the adhesive layers that are used to apply to skin, which show the sizes and shapes of the adhesive layers.
Figures 9A, 9B, and 9C are schematic, sectional views that show one example of a process for preparing the exothermic structure that is directly applied to skin according to this invention.
Figure 10 is a schematic, sectional view of one example of a portion for an exothermic body.
Figure 11 is a schematic, sectional view of one example of a portion for forming a partial adhesive layer.
Figure 12 is a schematic view of one example of a production line for the exothermic structure that is directly applied to skin according to this invention.
Figure 13 is a schematic view of another example of a production line for the exothermic structure that is directly applied to skin according to this invention.
Figure 14 is a schematic, sectional view of another example of the exothermic structure that is directly applied to skin according to this invention.

### DETAILED DESCRIPTION

Hereafter, this invention will be particularly explained with reference to figures that show preferable examples of this invention.

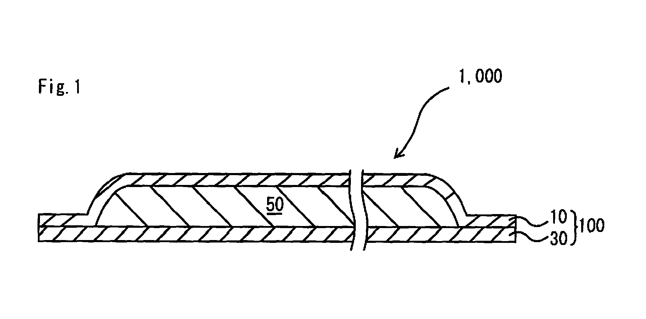

Figure 1 is a schematic, sectional view of a preferable example of the exothermic structure that is directly applied to skin according to this invention. The exothermic structure 1,000 that is directly applied to skin according to this invention comprises a pouch 100 having an air-permeable side 10 and an air-impermeable side 30, and an exothermic composition 50 that is put into the pouch 100 in a sealed state.

Figures 2A, 2B, and 2C are schematic, sectional views of preferable examples of the structures of the air-permeable side.

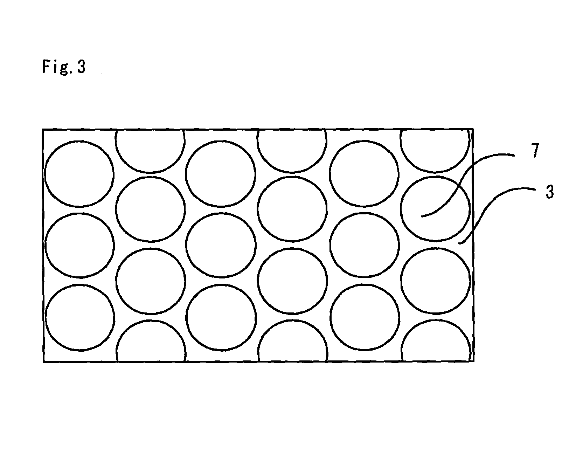

Figure 3 is a schematic, sectional view of another preferable example of the structure of the air-permeable side.

As shown in Figures 2A, 2B, and 2C, the air-permeable side is, for example, composed of an air-permeable polymer sheet 1, has a structure in which a fibrous layer such as non-woven fabric 3 is partially bonded to an air-permeable polymer sheet 1 with a bonding agent 5, or has a structure in which a fibrous layer such as non-woven fabric 3 and an air-permeable polymer sheet 1 are partially heat-sealed by having been partially and thermally fused the air-permeable polymer sheet 1. Or, as shown in Figure 3, a material of which air-permeability is controlled may be used as a material for the air-permeable side. The material has been made by partially coating a fibrous layer (an air-permeable material) such as a non-woven fabric 3 with an adhesive 7.

An example of the air-permeable polymer sheet 1 is an air-impermeable polymer sheet having air holes such as a moisture-permeable porous film made of a polyethylene. In this description, the term "moisture permeability" may be used. If moisture can pass through, a gas can also pass through. Namely, one having a moisture-permeability also has an air-permeability. If a moisture-permeable porous film is used as the air-permeable polymer sheet, its thickness is usually 100 µ m or less, preferably 20 to 80 µ m, and more preferably 40 to 60 µ m.

Examples of the fibrous layer that is used as a material to constitute the air-permeable side include non-woven fabric, woven fabric, knit, and paper.

If non-woven fabric is used, a spun lace, spun needle, or spunbonded non-woven fabric is preferred. Examples of materials of the non-woven fabric include rayons, nylons, polyesters, acrylics, polypropylene, Vinylon, polyethylene, urethanes, cotton, and celluloses. The non-woven fabric has a thickness of, as represented by the basis weight, usually 200 g/m² or less, preferably 20 to 120 g/m², and still more preferably 40 to 100 g/m².

If woven fabric is used, as materials of it, cotton, rayons, polyesters, polypropylenes, nylons, acrylics, and the like may be used. The woven fabric has a thickness of, as represented by the basis weight, usually 50 to 150 g/m², preferably 60 to 120 g/m², and still more preferably 70 to 100 g/m². If knit is used, as materials of it, rayons, polyesters, polypropylenes, nylons, acrylics, and the like may be used. The knit has a thickness of, as represented by the basis weight, usually 80 to 200 g/m², preferably 100 to 180 g/m², and still more preferably 120 to 150 g/m².

The air-permeability of the air-permeable side is regulated so that the exothermic composition 50 adequately generates heat. It is preferable to select and process materials that constitute the air-permeable side so that the air-permeable side has a moisture-permeability [JIS K 7129(2008)] of 200 to 500 g/m² 24 hours (preferably 250 to 400 g/m² 24 hours) which is determined by the Lyssy method. Methods for processing materials that constitute the air-permeable side so that the air-permeable side has a desirable air-permeability or moisture-permeability, for example, a method for controlling the air-permeability or the moisture-permeability by a bonding process when a laminate is used and a method for preparing a porous film having a desirable air-permeability or moisture-permeability, have been known.

The exothermic composition 50 is constituted of a composition comprising a component that generates heat by oxygen. The components that are contained in the exothermic composition are not particularly limited as long as they have been conventionally used in the exothermic compositions that generate heat by air. Examples of the components are as follows.

Examples of chemical exothermic agents include metal powders such as iron powders (reduced iron powder, atomized iron powder, and the like). Examples of reaction auxiliaries include metal halides such as sodium chloride, potassium chloride, magnesium chloride, calcium chloride, iron (II) chloride, and iron (III) chloride; metal sulfates such as potassium sulfate, sodium sulfate, magnesium sulfate, copper sulfate, iron (II) sulfate, and iron (III) sulfate. Examples of water retaining agents include active carbon, alumina, silica gel, zeolite, wood charcoal, and water-absorptive polymeric compounds. Of course, water is also used. Examples of other additives include polymeric compounds such as carboxymethyl cellulose, acrylic acid starch, polyethylene, polypropylene, and polystyrene; bentonite; vermiculite; pearlite; and wood charcoal.

It is preferable that an exothermic composition having a formula is used by which formula a metal powder such as an iron powder has a less tendency of deflection. The exothermic composition is preferably processed so as to be a sheet-like form. In this case the thickness is preferably 5 mm or less, still more preferably 0.5 to 4 mm, and particularly preferably 1 to 2 mm.

The exothermic structure that is directly applied to skin according to this invention is characterized in the constitution of the air-impermeable side 30 of the pouch 100. The constitution of the air-impermeable side 30 will be specifically explained by referring to figures.

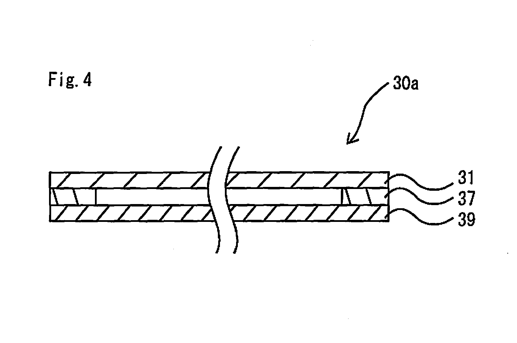

Figure 4 is a sectional view of one example of the air-impermeable side. The air-impermeable side 30a has an inner (namely, the side that contacts the exothermic composition 50) polymer sheet layer 31 and an outer release sheet 39, and further has an adhesive layer 37 that is used to directly apply to skin on an inner surface of the release sheet 39 in its outer periphery.

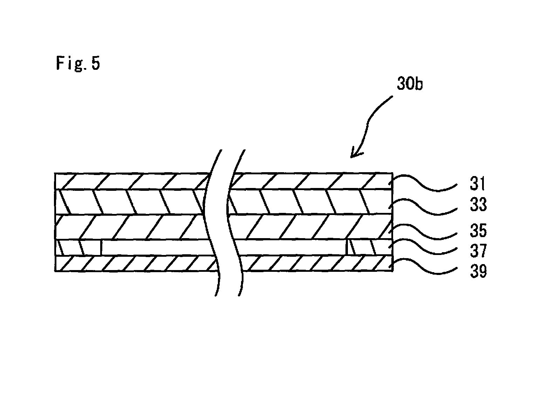

Figure 5 is a sectional view of another example of the air-impermeable side. The air-impermeable side 30b has, from inside (namely, the side that contacts the exothermic composition 50) to outside, a polymer sheet layer 31, a layer 33 of a tackiness agent, a fibrous layer 35, and a release sheet 39 in this order, and further has an adhesive layer 37 that is used to directly apply to skin on an inner surface of the release sheet 39 in its outer periphery. The layer 33 is made entirely on the outer surface (namely, the side that does not contact the exothermic composition 50) of the polymer sheet layer 31.

Examples of polymeric materials for the polymer sheet layer 31 include polyolefins such as polyethylene and polypropylene; polyamides such as nylons; polyesters such as polyethylene terephthalates; ethylene copolymers such as ethylene-vinyl acetate copolymers and their saponified ones, and ethylene-alkyl (meth)acrylate copolymers; poly(vinyl chloride); poly(vinylidene chloride); polyurethanes; and polystyrenes. The polymer sheet is usually air-impermeable. However, in an embodiment wherein the layer 33 of the tackiness agent is made entirely on the outer surface (namely, the side that does not contact the exothermic composition 50) of the polymer sheet layer 31, a polymer sheet having an air-permeability to an extent can be used.

In this description, the terms "polymer sheet" and "polymer film" are exchangeable.

Specific examples of the air-impermeable polymer film that constitutes the polymer sheet layer 31 include polyethylene films. Among them because of their excellent heat-sealability, linear low-density polyethylene films and metallocene polyethylene films are preferable. These polymer films have a thickness of usually100 µm or less, preferably 10 to 70 µm, more preferably 20 to 50 µm, and still more preferably 25 to 45 µm. The polymer film that constitutes the polymer sheet layer 31 is not limited to a mono-layered one, but may be a multi-layered one. Also, processed films such as films on which a metal or an inorganic material has been vapor-deposited may be used.

Examples of tackiness agent compositions that are used to form the layer 33 comprising a tackiness agent include rubber-type ones, acryl-type ones, silicone-type ones, and those that comprise as the main component thermoplastic resins such as polyamide resins, polyethylene resins, and cellulose resins.

Examples of tackiness agents that are used in the rubber-type tackiness agent compositions include diene-type polymeric compounds such as natural rubbers, synthetic rubbers, and mixtures of them. Examples of synthetic rubbers include styrene-isoprene block copolymer rubber, styrene-isoprene-styrene block copolymer rubber, styrene-isobutylene-styrene block copolymer rubber, styrene-butadiene rubber, polyisoprene rubber, butyl rubber, chloroprene rubber, nitrile rubber, polysulfide rubber, and silicone rubber.

Examples of tackiness agents that are used in the acryl-type tackiness agent compositions include conventionally-used copolymers of at least one (meth)acrylate such as n-butyl (meth)acrylate, hexyl (meth)acrylate, decyl (meth)acrylate, dodecyl (meth)acrylate, and tridecyl (meth)acrylate, with a functional monomer that is copolymerable with the (meth)acrylate such as (meth)acrylic acid, maleic acid, maleic anhydride, hydroxyethyl acrylate, hydroxypropyl acrylate, acrylamide, dimethylacrylamide, aminoethyl methacrylate, and methoxyethyl (meth)acrylate or a vinyl monomer that is copolymerable with the (meth)acrylate such as acrylonitrile, vinyl acetate, and vinyl propionate.

The condition of the tackiness agent composition is not particularly limited as long as it can be readily applied onto the surface of the polymer film, for example, an emulsion, a solution with a solvent, an aqueous solution, or a hot-melt type one. If the inner-most layer of the air-permeable side 10 is heat-sealed with the polymer sheet layer 31 that is the inner-most layer of the air-impermeable side 30, the tackiness agent composition of the hot-melt type is inappropriate. This is because the layer 33 fuses during heat-sealing. If the heat-sealing is done, it is preferable that the tackiness agent that constitutes the layer 33 is excellent in heat resistance. From these points of view, it is preferable to prepare the layer 33 by using an acrylic pressure-sensitive adhesive composition of a solvent, aqueous-solvent, or emulsion type, which gives a pressure-sensitive adhesive layer.

Examples of materials that constitute the fibrous layer 35 include non-woven fabric, woven fabric, knit, and paper.

If non-woven fabric is used, a spun lace, spun needle, or spunbonded non-woven fabric is preferred. Examples of materials of the non-woven fabric include rayons, nylons, polyesters, acrylics, polypropylene, Vinylon, polyethylene, urethanes, cotton, and celluloses. The non-woven fabric has a thickness of, as represented by the basis weight, usually 200 g/m² or less, preferably 20 to 120 g/m², and still more preferably 40 to 100 g/m².

If woven fabric is used, as materials of it, cotton, rayons, polyesters, polypropylenes, nylons, acrylics, and the like may be used. The woven fabric has a thickness of, as represented by the basis weight, usually 50 to 150 g/m², preferably 60 to 120 g/m², and still more preferably 70 to 100 g/m². If knit is used, as materials of it, rayons, polyesters, polypropylenes, nylons, acrylics, and the like may be used. The knit has a thickness of, as represented by the basis weight, usually 80 to 200 g/m², preferably 100 to 180 g/m², and still more preferably 120 to 150 g/m².

In the exothermic structure according to this invention, the fibrous layer 35 plays a role of conveying heat that has been generated by the exothermic composition to human body, another role of absorbing liquids such as sweat and the like, and still another role as a support for maintaining the form of the exothermic structure. From these points of view, as the fibrous layer 35 non-woven fabric is preferably used and non-woven fabric comprising both a hydrophilic fiber and a hydrophobic fiber is more preferably used. This is because the hydrophilic fiber is excellent in a property of absorbing liquids such as sweat and the hydrophobic fiber is excellent in a heat-conductivity Because the heat-conductivity of the layer of the non-woven fabric is reduced when the hydrophilic fiber absorbs a liquid such as sweat, an effect is enhanced that a burn at low-temperatures is prevented. In consideration of these characteristics of the hydrophilic fiber and the hydrophobic fiber, a mixture comprising the hydrophilic fiber and the hydrophobic fiber at a ratio in the range of 10: 90 to 70 : 30 (weight ratio) is preferably used, a mixture comprising those fibers at a ratio in the range of 20 : 80 to 70 : 30 (weight ratio) is more preferably used, and a mixture comprising those fiber at a ratio in the range of 30 : 70 to 50 : 50 (weight ratio) is particularly preferably used as the materials for the non-woven fabric.

Examples of the hydrophilic fiber include natural fibers such as cotton, wool, silk, hemp, and wood pulp; cellulose fibers such as rayon and cupra; poly(vinyl alcohol) fibers; cellulose-acetate fibers; and highly water-absorbable fibers (for example, crosslinked acrylate fibers, processed acrylic fibers of which surfaces have been hydrolyzed, fibers that have been obtained by graft-polymerizing acrylic acid or methacrylic acid to fibers of polyesters and the like, etc.). Examples of the hydrophobic fiber include fibers that are made of polyesters, nylons, acrylics, and the like.

The adhesive layer 37 that is used to directly apply to skin is formed on an inner surface of the release sheet 39 in the outer periphery. Examples of the adhesive composition that are used to form the adhesive layer 37 include rubber-type adhesive compositions, acrylic-type adhesive compositions, silicone-type type adhesive compositions, and other adhesive compositions each comprising as the main component a thermoplastic resin (for example, a polyaminde-type resin, a polyethylene-type resin, or a cellulose-type resin).

The adhesive layer 37 is used to apply to skin. Thus, it is preferable to use materials that are no or low-irritative to skin. From this point of view, rubber-type adhesive compositions are preferably used. Specific examples of the adhesives that are used in the rubber-type adhesive compositions and the acrylic-type adhesive compositions are the same as those that have been explained about the tackiness agents in the layer 33.

The surface of the adhesive layer 37 is covered with the release sheet 39. The release sheet 39 covers not only the surface of the adhesive layer 37, but also the entire surface of the polymer sheet layer 31 (in the embodiment shown in Figure 4) or the fibrous layer 35 (in the embodiment shown in Figure 5). Namely, the release sheet 39 has a shape and size that are the same as those of the polymer sheet layer 31 or the fibrous layer 35.

The materials for the release sheet 39 are not limited as long as they have been conventionally used for the sheet for covering the adhesive layer of the exothermic structure, namely, for the release sheet. For example, various plastic films, metal foils, and a laminate of a plastic film and paper can be used as the release sheet. A coating agent for release, such as a silicone type one, an alkylacrylate type one, or a fluorine type one, may be applied onto the release sheet. Examples of polymeric compounds that constitute the plastic films include polyesters, polypropylene, polyethylene, alkylbenzene sulfonates, poly(vinyl chloride), and fluorinated ethylene-propylene copolymer.

In the exothermic structure 1,000 that is directly applied to skin according to this invention, the adhesive layer 37 that constitutes the air-impermeable side of the pouch 100 has following features:
(1) the adhesive layer is formed in part or entire of an outer periphery (preferably, only a portion that corresponds to a portion where the air-permeable side and the air-impermeable side are bonded together, namely, only a portion where there are no exothermic composition) on an inner surface of the release sheet;
(2) the total area of the adhesive layer is from 10 to 40% (preferably 10 to 35%, more preferably 10 to 30%) of the area of the release sheet;
(3) the adhesive layer has an adhesive portion(s) and a non-adhesive portion(s) or only an adhesive portion(s);
(4) the total area of the adhesive portion(s) is from 30 to 100% (preferably 40 to 100%, more preferably 50 to 100%) of the total area of the adhesive layer; and
(5) the total length of the adhesive portion(s) is from 60 to 100% (preferably 70 to 100%, more preferably 80 to 100%) of the total length of the adhesive layer in the outer periphery.

The terms "outer periphery" mean an outer portion. In this invention, the terms "outer periphery" mean, approximately, part or entire of the portion that corresponds to the portion where the air-permeable side and the air-impermeable side are bonded together, namely, to the portion where there are no exothermic composition. The outer periphery may also include part of the portion that corresponds to the portion where there is an exothermic composition.

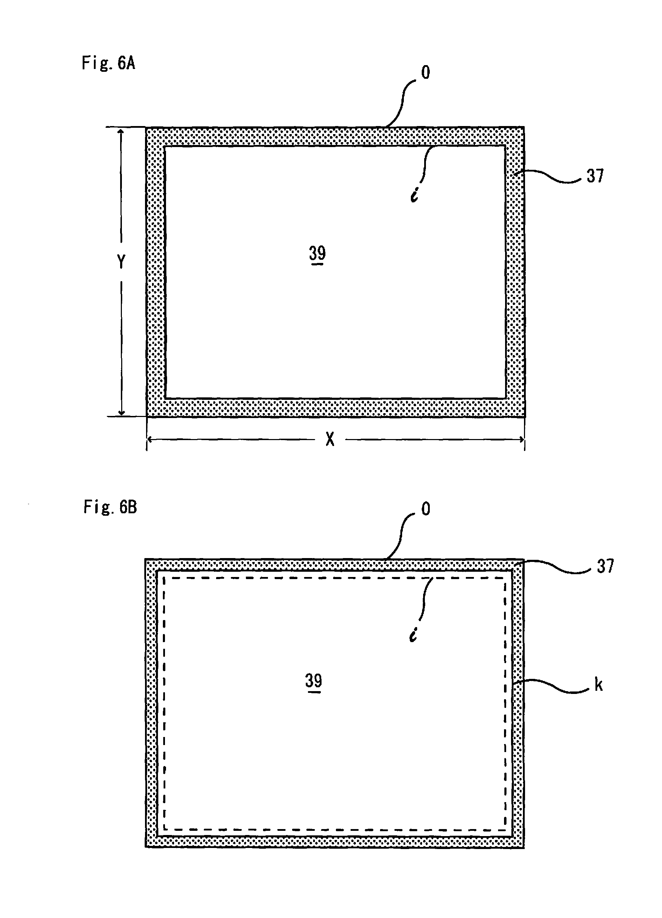

Based on Figures 6A and 6B, the above items (1) and (2) will be explained. Figures 6A and 6B are schematic, plane views of the inner surface of the release sheet 39.

In Figures 6A and 6B the numeral i shows the inner circumference of the portion that corresponds to the portion where the air-permeable side 10 and the air-impermeable side 30 are bonded together (namely, the portion where there is no exothermic composition). The numeral o shows the outer circumference of the portion that corresponds to the portion where the air-permeable side 10 and the air-impermeable side 30 are bonded together. In this case, the outer circumference o is also the outer circumference of the exothermic structure. The term "bonding" means, e.g., heat-seal or adhesion with an adhesive.

In Figures 6A and 6B, the portion where the adhesive layer is formed is shown by numerous dots, namely, by the portion 37. In the adhesive layer the adhesive portions are no limited to have a polka-dot pattern (hereafter the same may be applied). In the example as shown in Figure 6A, the adhesive layer 37 is formed in entire of the outer periphery and in entire and only the portion corresponding to the portion where the air-permeable side 10 and the air-impermeable side 30 are bonded together on the inner surface of the release sheet 39.

In the example as shown in Figure 6B, the adhesive layer 37 is formed in part of the portion corresponding to the portion where the air-permeable side 10 and the air-impermeable side 30 are bonded together. Specifically, the adhesive layer 37 has its inner circumference at the position k, wherein the position k exists outside the inner circumference i (in other words, between the inner circumference i and the outer circumference o) of the portion corresponding to the portion where the air-permeable side 10 and the air-impermeable side 30 are bonded together. In this example, the adhesive layer 37 is formed in entire of the outer periphery but in part of the portion corresponding to the portion where the air-permeable side 10 and the air-impermeable side 30 are bonded together on the inner surface of the release sheet 39.

The total area of the adhesive layer 37 is from 10 to 40% of the total area (i.e., X x Y) of the release sheet 39.

The width or distance between the inner circumference and the outer circumference, namely, in the case shown in Figure 6A the width or distance between the inner circumference i and the outer circumference o, and in the case shown in Figure 6B the width or distance between the inner circumference k and the outer circumference o, is preferably from 5 to 30 mm.

In the examples as shown in Figures 6A and 6B, the adhesive layer 37 is formed only the portion corresponding to the portion where the air-permeable side and the air-impermeable side are bonded together. However, if the portion is in an outer periphery, the adhesive layer 37 may also be formed in part of the portion corresponding to the portion where there is an exothermic composition.

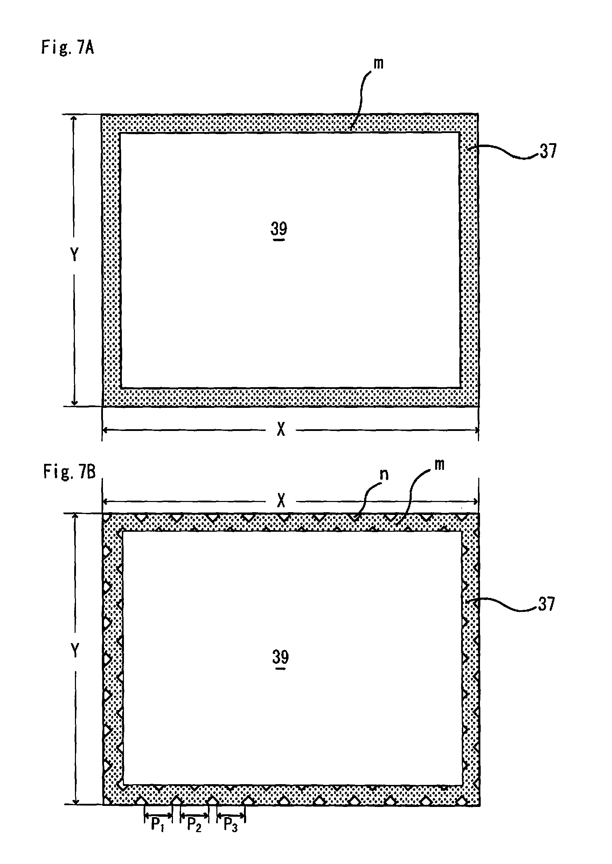

Next, based on Figures 7A and 7B, the above items (3) to (5) will be explained. Figures 7A and 7B are schematic, plane views of the release sheet 39. Figure 7A shows an example in which the adhesive layer 37 comprises only an adhesive portion and Figure 7B shows an example in which the adhesive layer 37 comprises adhesive portion m and non-adhesive portions n. The adhesive portion m is shown with numerous dots and is distinguished from the non-adhesive portions n in Figure 7B.

In the example as shown in Figure 7A, the ratio of the adhesive portion m in the adhesive layer 37 is 100%. In the example as shown in Figure 7B, the ratio of the adhesive portion m in the adhesive layer 37 is about 80%. In the example as shown in Figure 7A, 100% of the total length (2X + 2Y) of the adhesive layer 37 in the outer periphery is derived from the adhesive portion m.

In the example as shown in Figure 7B, in the total length (2X + 2Y) of the adhesive layer 37 in the outer periphery, the portions that are derived from the adhesive portion m are (P₁ + P₂ + P₃ ...... + Pₙ) and the ratio of the portions that are derived from the adhesive portion m is about 67%. Because the non-adhesive portions n also constitute the adhesive layer 37, in this example the adhesive layer 37 is formed in entire of the outer periphery.

The shape of the non-adhesive portions n is not limited. One example is diamond as shown in Figure 7B. In other words, the shape is a diagonal check wherein the adhesive portion m has a width on some level. The concept of the diamond comprises square. It is preferable that in the four sides of the outer periphery in the adhesive layer 37, the ratios that are derived from the adhesive portion m are almost equal. An exothermic structure having such a constitution is difficultly peeled off during its use.

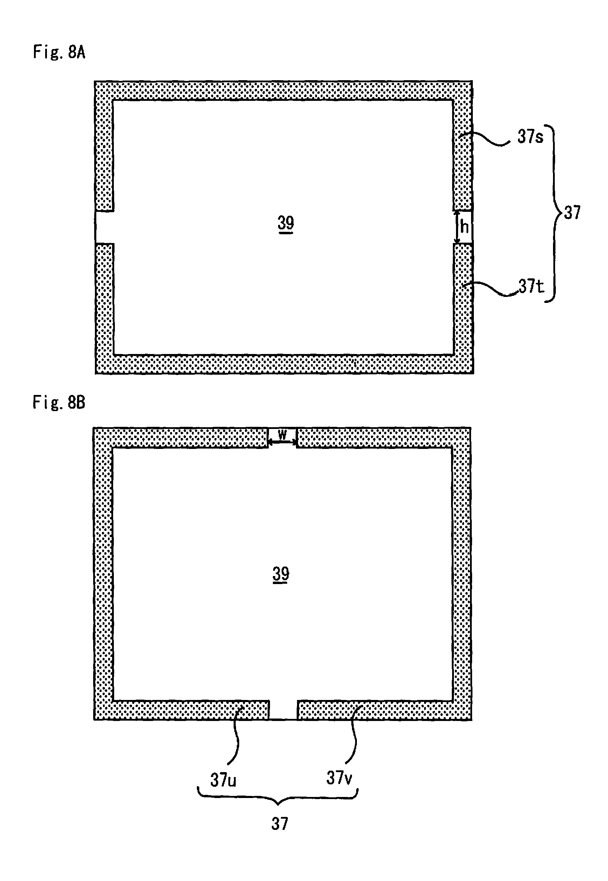

Figures 8A and 8B show examples wherein the adhesive layer is formed in part of the outer periphery on the release sheet 39 and in only part of the portion corresponding to the portion where the air-permeable side and the air-impermeable side are bonded together. Figures 8A and 8B are schematic, plane views each showing the inner surface of the release sheet 39. In Figures 8A and 8B, the portions 37s, 37t, 37u, and 37v that constitute the adhesive layer 37 are shown with numerous dots. The adhesive layer 37 may have an adhesive portion(s) and a non-adhesive portion(s), or only an adhesive portion(s). The position of the inner circumference of the adhesive layer 37 may the position that is the same as the position i of the inner circumference of the portion corresponding to the portion where the air-permeable side 10 and the air-impermeable side 30 are bonded together (see Figure 6A). The position of the inner circumference of the adhesive layer 37 may the position k that is outside the position i, namely, that is between the inner circumference i and the outer circumference o (see Figure 6B). Alternatively, the adhesive layer 37 may also be formed in part of the position corresponding to the position where there is an exothermic composition.

In the example as shown in Figure 8A, the adhesive layer 37 is formed to have two U-like-shaped portions 37s and 37t that are axisymmetric to each other, and the distance h between the U-like-shaped portions where there is no adhesive layer 37 is from 10 to 30 mm. In the example as shown in Figure 8B, the adhesive layer 37 is formed to have two U-like-shaped portions 37u and 37v that are axisymmetric to each other, and the distance w between the U-like-shaped portions where there is no adhesive layer 37 is from 10 to 30 mm. By inserting a top of a finger or a nail into the portion where the adhesive layer 37 lacks, the release sheet 39 can be readily removed.

The exothermic structure that is directly applied to skin according to this invention is kept in an outer bag. The outer bag is constituted of a moisture resistant, air-impermeable material. Because the outer bag is air-impermeable, the exothermic agent that generates heat by air in the exothermic composition does not chemically react, and thus the exothermic composition is kept without generating heat. After the outer bag is opened, air (oxygen) gets to the exothermic agent through the air-permeable side of the pouch of the exothermic structure. Then, the chemical reaction starts and heat of reaction is emitted.

A representative example of the material for the outer bag is a laminate of aluminum foil with a polymer film.

Next, a process for preparing an exothermic structure that is directly applied to skin according to the present invention will be explained with referring to Figures.

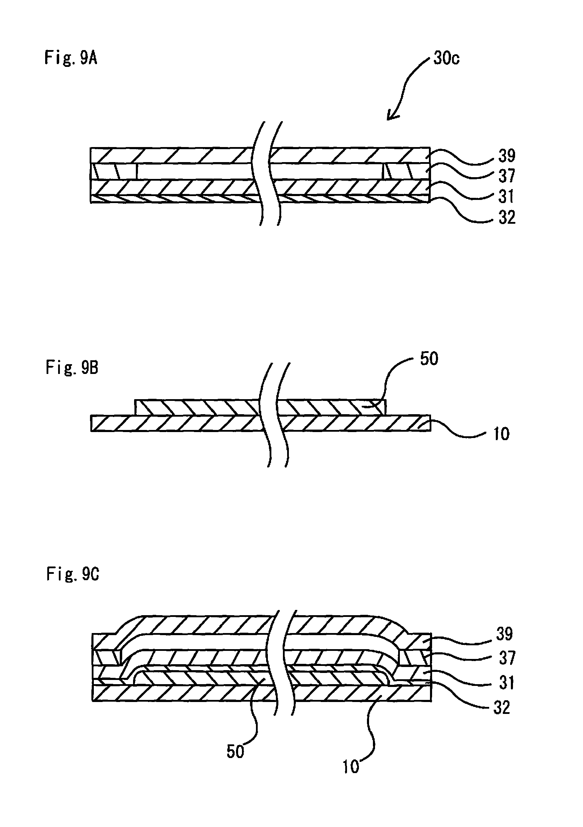

Figures 9A, 9B, and 9C are schematic, sectional views that show one example of a process for preparing the exothermic structure that is directly applied to skin according to this invention. In this process, first, the structure 30c as shown in Figure 9A is prepared. Namely, an adhesive layer 37 is formed on one surface of the release sheet 39 in outer periphery, wherein the surface of the adhesive layer 37 is covered with a polymer sheet layer 31. Then, onto entire of the outer surface of the polymer sheet layer 31, a bonding agent is applied to give a layer 32. Thus, a material for an air-impermeable side can be obtained. Figure 9A shows a schematic, sectional view of an air-impermeable side 30c of one exothermic structure having one pouch. Usually, in the material for the air-impermeable side, numerous portions that correspond to air-impermeable sides 30c of the exothermic structures are connected.

Separately, as shown in Figure 9B, on one surface of the air-permeable side 10 an exothermic composition 50 is put. Next, as shown in Figure 9C, the surface of the exothermic composition 50 is covered with a material for air-impermeable side 30c. At this time the side of the layer 32 of the bonding agent faces the air-permeable side 10. The bonding agent may be applied onto only the outer periphery (namely, the portion corresponding to the portion where there is no exothermic composition 50; in other words, the portion that is used to bond to the air-permeable side 10) on the outer surface of the polymer sheet layer 31.

The type of the bonding agent for the layer 32 is not limited. For example, an acrylic emulsion type is preferable.

By using the layer 32 of the bonding agent, the air-permeable side 10 is bonded to the air-impermeable side 30c. If one is produced wherein numerous exothermic structures are connected, at the end cut is done at the center of the width of the portion where there is the adhesive layer 37 and that constitutes parts of two exothermic structure portions to give each exothermic structure. The structure thus obtained is put into an air-proof outer bag in a sealed state.

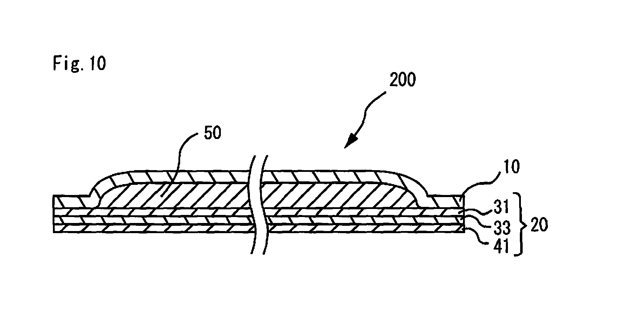

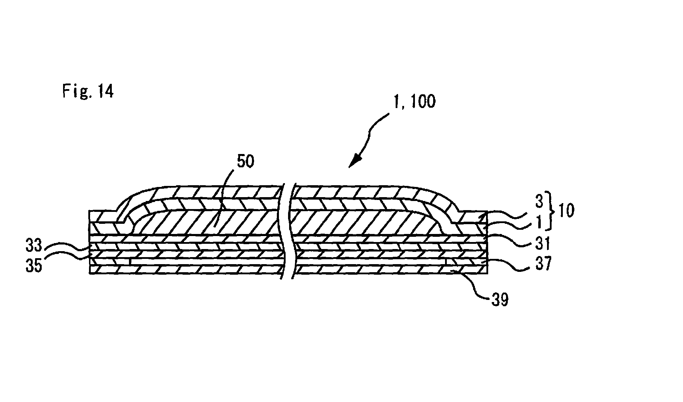

Next, by referring to Figures 10 to 14 the process for preparing an exothermic structure 1,100 that is directly applied to skin will be explained. The exothermic structure 1,100 is shown in Figure 14 and has an air-impermeable side 30b as shown in Figure 5.

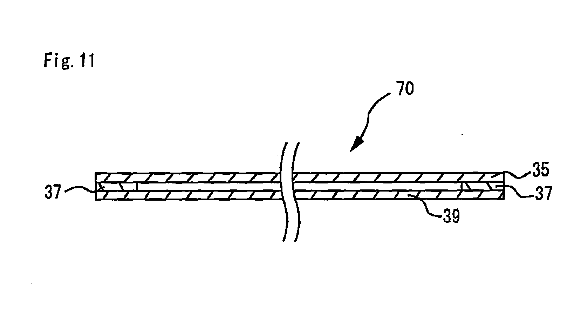

To produce the exothermic structure 1,100, first a portion 200 for an exothermic body as shown in Figure 10 and a portion 70 for forming a partial adhesive layer as shown in Figure 11 are separately produced.

First, a first sheet 10x as a material for an air-permeable side 10 is prepared. In this example, the first sheet 10x has a constitution wherein a fibrous layer such as a non-woven fabric 3 and an air-permeable polymer sheet 1 is partly bonded together with a bonding agent 5 as shown in Figure 2B or another constitution wherein a fibrous layer such as a non-woven fabric 3 and an air-permeable polymer sheet 1 is partly heat-sealed as shown in Figure 2C.

Separately, a second sheet 20x is prepared. The second sheet 20x is a material for a constitution 20 wherein on one surface of a polymer sheet 31 a layer 33 of a tackiness agent is made and the surface of the layer 33 is covered with a release paper 41.

Further, the portion 70 for forming a partial adhesive layer as shown in Figure 11 is produced. Namely, an adhesive layer 37 is formed by, e.g., applying an adhesive composition in a portion corresponding to the portion that would be the adhesive layer of the exothermic structure on one surface of the release sheet 39. Then, the adhesive layer 37 is transferred to a material for the fibrous layer, e.g., a non-woven fabric 35, specifically, the adhesive layer 37 is adhered to the fibrous layer by press, to give the portion 70 for forming partial adhesive layer. At this time part of the adhesive layer 37 penetrates into the material for the fibrous layer.

On the surface of the release sheet 39 the adhesive layer 37 may be formed by using a coater of, e.g., a gravure, reverse, screen printing, or other coater printing system.

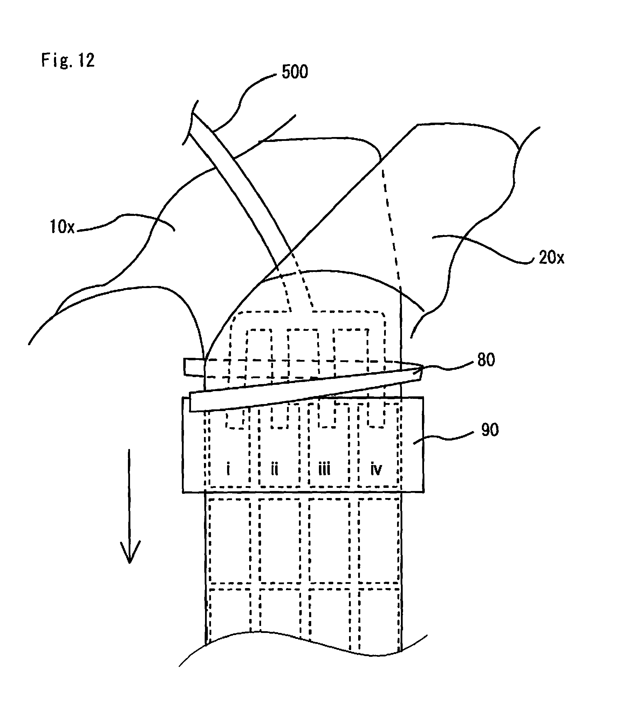

The exothermic structure is produced as follows: First, as shown in Figure 12 the first sheet 10x and the second sheet 20x are set so that the one that faces the exothermic composition 50 or is to be heat-sealed in the first sheet 10x (i.e., the material for the air-permeable side 10) faces the polymer sheet 31 of the second sheet 20x. Then, heat-sealing in a transverse direction is done with a transverse heat-sealer 80. The sheets are gone forward to the traveling direction (the direction of the arrow). When the transverse heat-sealed portion arrives at the downstream of the longitudinal heat-sealers 90, the longitudinal heat-sealing is done to give four pouch portions i, ii, iii, and iv in a transverse line, wherein each pouch portion has three heat-sealed sides. The longitudinal heat-sealing may be done during the sheets are going forward to the traveling direction (the direction of the arrow).

The exothermic composition is put into the pouch portions i, ii, iii, and iv with a filling tube 500 for the exothermic composition. Then, by using the transverse heat-sealer 80 the transverse heat-sealing is done to close the pouch portions i, ii, iii, and iv. Thus, one having four exothermic body portions 200, 200, 200, and 200 in a transverse line can be obtained. Thereafter, the release paper 41 is removed.

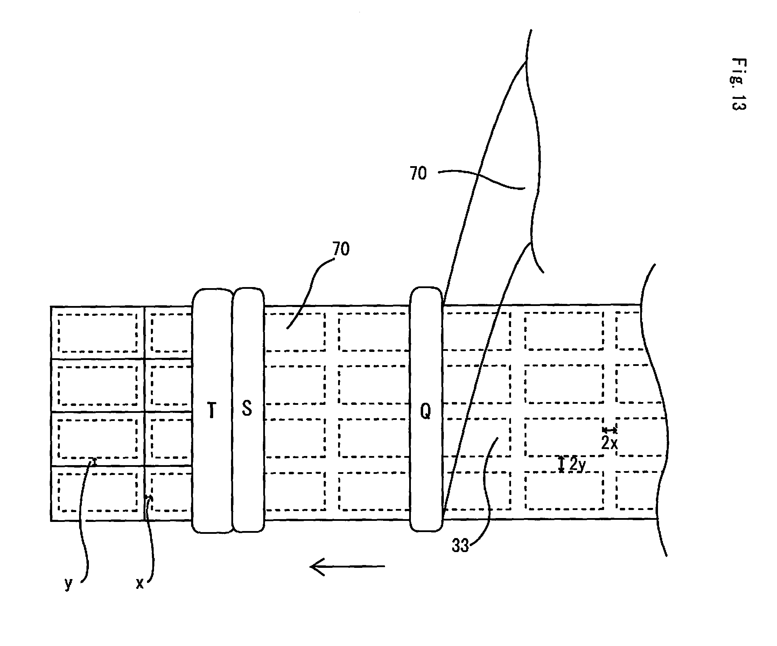

Next, as shown in Figure 13 onto the layer 33 of the tackiness agent that has been bared by removing the release paper 41, a portion 70 for forming a partial adhesive layer is applied by pressing with a press roller Q, wherein one side of the material for the fibrous layer (for example, non-woven fabric 35) where there is no adhesive layer 37 in the portion 70 faces the layer 33. By using a cutter S for longitudinal heat-sealed portions, e.g., a rotary blade, the longitudinal heat-sealed portions are cut at the center of the width 2y. By using a cutter T for a transverse heat-sealed portion, e.g., a roll cutter, the transverse heat-sealed portion is cut at the center of the width 2x. Thus, an exothermic structure 1,100 is obtained. Then, the exothermic structure 1,100 is put into an air-proof outer bag in a sealed state.

The process for producing the exothermic structure according to this invention is not limited to the above process. For example, instead of the heat-sealing an adhesion with a bonding agent may be conducted. By using, e.g., a die-cut apparatus having a Thomson-type blade, cut in the longitudinal heat-sealed portions and cut in the transverse heat-sealed portion may be simultaneously done.

### EFFECT OF INVENTION

In the exothermic structure that is directly applied to skin, the adhesive layer that is used to apply to skin exists only the periphery (specifically, the outer periphery) of the structure. Therefore, the portion where there is an exothermic composition rarely close-contacts with skin, and low-temperature burn rarely occurs. Specifically, in the case where the adhesive layer is formed only on the portion where there is no exothermic composition (namely, the portion that corresponds to the portion where the air-permeable side and the air-impermeable side are bonded together), another portion where there is the exothermic composition does not closely contact with skin. Thus, the risk of the low-temperature burn is lower.

The exothermic structure that is directly applied to skin according to this invention has the adhesive layer that is used to apply to skin only the periphery (specifically, the outer periphery) of the structure, the total area of the adhesive layer is from 10 to 40 % of the total area of the release sheet, the adhesive layer has an adhesive portion(s) and a non-adhesive portion(s) or only an adhesive portion(s), the total area of the adhesive portion(s) is from 30 to 100% of the total area of the adhesive layer, and the total length of the adhesive portion(s) is from 60 to 100% of the total length of the adhesive layer in the periphery. Thus, during its use, the structure is difficultly peeled off.

If the distance between the outer periphery and the inner periphery of the adhesive layer is from 5 to 30 mm, the above two effects are enhanced.

If the adhesive layer also has a non-adhesive portion(s), the risk of the low-temperature burn is still more lowered.

If the shape of the non-adhesive portion(s) is diamond, in other words, the adhesive portions are in the form of check, the effect that the structure is difficultly peeled off is enhanced.

If the adhesive layer has two U-like-shaped portions that are axisymmetric to each other, and the distance between the U-like-shaped portions is from 10 to 30 mm, the release sheet can be readily removed.

According to the process for preparing an exothermic structure that is directly applied to skin of this invention, the adhesive layer is not suffered from a heat treatment such as a heat-sealing. Thus, for the adhesive layer, every adhesive can be used. Thus, as the adhesive, no or a mildly irritating one can be selected without the need of considering its thermal resistance (especially a melt temperature).

According to the process for preparing an exothermic structure that is directly applied to skin of this invention, plural exothermic structures can be simultaneously produced with high dimensional accuracy. Thus, the production efficiency is very high.

Further, according to the process for preparing an exothermic structure that is directly applied to skin of this invention, only by controlling the cutting intervals different exothermic structures (for example, an exothermic structure having only one pouch portion and other exothermic structure having two or more pouch portions) can be made by using one production machine.

Hereafter this invention will be specifically explained in reference to examples. However, it is not intended that this invention is limited to the examples.

### EXAMPLE 1

### (Preparation of Exothermic Composition)

The exothermic composition was prepared according to the formula as shown in Table 1 by an ordinary method.

**TABLE 1**

| Name of materials | Formulation (% by weight) |
|---|---|
| Iron powder | 60 |
| Active carbon | 5 |
| Carboxymethyl cellulose | 2 |
| Acrylic acid starch | 2 |
| Sodium chloride | 2 |
| Tap water | 29 |
| Total | 100 |

### (Production of Exothermic Structure 1)

The exothermic structure 1,100 that is shown in Figure 14 and has an adhesive layer that is directly applied to skin of Figure 6A was produced as follows:
(1) A porous film 1 made of a polyethylene (manufactured by KOUJIN CO., LTD.; TSF-EU; a thickness of 50 µm) was partly bonded to a spun lace non-woven fabric 3 made of a polyester (manufactured by ASAHI KASEI CORPORATION; a basis weight of 60 g/m²). Thus, a sheet (the first sheet 10x) for an air-permeable side 10 was obtained. The moisture permeability (JIS K 7129(2008)) of the first sheet 10x was 310 g/m². 24hours by the Lyssy method.
(2) An acrylic solvent adhesive (manufactured by NIPPON SYNTHETIC CHEMICAL INDUSTRY CO., LTD.; CORPONORL 5411) was applied onto one side of a commercially available air-impermeable polyethylene film 31 (manufactured by FUKUI MINASELL CO., LTD.; a thickness of 40 µm) entirely so as to have a thickness of 0.025 mm in a dry state. By heat-drying, a layer 33 of a tackiness agent was made. The surface of the layer 33 was covered with a commercially available release paper 41 (manufactured by DAIO PAPER CONVERTIMNG CO., LTD.; Blue glassine paper). Thus, the second sheet 20x (corresponding to a portion as shown in numeral 20 in Figure 10) was prepared.
(3) The first sheet 10x and the second sheet 20x were set so that the porous film 1 faces the air-impermeable polyethylene film 31, and were laterally heat-sealed with a transverse heat-sealer. Then, the first sheet 10x and the second sheet 20x were carried forward. After the laterally heat-sealed portion came to an end of a longitudinal heat-sealer, the sheets were longitudinally heat-sealed with five longitudinal heat-sealers to form four pouch portions. The exothermic composition having a formula of Table 1 was put into every pouch portion in an amount of 20g per pouch portion (see Figure 12).
(4) Again, the sheets were laterally heat-sealed. The pouch portions into which the exothermic composition had been put were pressed so that the exothermic composition would have a thickness of about 1.5 mm. Thus, an exothermic portion 200 as shown in Figure 10 was produced.
(5) Separately, on one surface of a polyester film 39 having a silicone-coating layer (manufactured by TOYO METALLIZING CO., LTD., Cerapeel; a thickness of 38 µ m) that would be used as a release sheet, an adhesive of a styrene-isoprene-styrene block-copolymer type was applied so that an adhesive layer 37 that would be directly applied to skin for one exothermic structure would be made at a portion corresponding to the laterally and longitudinally heat-sealed portions in step (3) in a same size (see figure 6A). Then, the adhesive was dried to form the adhesive layer 37 that is directly applied to skin.
(6) The surface of the adhesive layer 37 was covered with a spun lace non-woven fabric 35 made of a polyester/rayon (80:20 by weight) (manufactured by ASAHI KASEI CORPORATION; a basis weight of 40 g/m²) having a size that was the same as that of the polyester film 39. Then, the adhesive layer 37 was transferred onto the spun lace non-woven fabric 35. Thus, portions for forming partial adhesive layers were formed. Figure 11 shows a portion 70 that corresponds to a portion for forming a partial adhesive layer for one exothermic structure.
(7) The release paper 41 of the exothermic body portion 200 was removed, and the portion 70 for forming the partial adhesive layer was applied onto the bared layer 33 of the tackiness agent. The side of the spun lace non-woven fabric 35 that did not have the adhesive layer 37 was applied onto the layer 33 (see Figure 13).
(8) At the center in width ("2y" in Figure 13) of each longitudinally heat-sealed portion cut was done with a rotary blade, and then at the center in width ("2x" in Figure 13) of the laterally heat-sealed portion cut was done with a rolling cutter. Thus, a exothermic structure 1,100 was obtained. The structure 1,100 was put into an airproof outer bag in a sealed state.

The exothermic structure 1,100 (production example 1) thus produced had a size of 9.5 cm x 13 cm and the width (same as the widths of the heat-sealed portions, namely, "x" and "y" in Figure 13) of the adhesive layer 37 that is directly applied to skin in the outer periphery was 8 mm.

### (Production of Exothermic Structure 2)

The exothermic structure (production example 2) was produced in the same manner as that of the production of the exothermic structure 1, except that the adhesive layer 37 that is directly applied to skin had an adhesive portion having a shape of an oblique check as shown in Figure 7B, wherein the width of the check was 4 mm and the width of each non-adhesive portion was 2 mm.

### (Production of Exothermic Structure 3)

The exothermic structure (production example 3) was produced in the same manner as that of the production of the exothermic structure 1, except that the width of the adhesive layer 37 that is directly applied to skin was 4 mm as shown in Figure 6C.

Table 2 shows, for production examples 1 to 3, the total area of the adhesive layer/the total area of the release sheet, the total area of the adhesive portions/the total area of the adhesive layer, and the total length of the adhesive portions/the total length of the adhesive layer in the outer periphery.

**TABLE 2**

| | Total area of adhesive layer 37 / total area of release sheet 39 | Total area of adhesive portions / total area of adhesive layer 37 | Total length of adhesive portions / total length of adhesive layer 37 in the outer periphery |
|---|---|---|---|
| Production example 1 | 27.1% | 100% | 100% |
| Production example 2 | 27.1% | About 80% | About 67% |
| Production example 3 | 14.1% | 100% | 100% |

### (Test Example)

A use test of the exothermic structure was conducted. One group consisted of ten subjects. Specifically, there were two subjects that evaluated by themselves to have much body hair, three subjects that evaluated by themselves to have some body hair, and five subjects that evaluated by themselves to have body hair in a normal amount.

One exothermic structure was applied onto a lower right arm (i.e., between right wrist and right elbow) of a subject and another exothermic structure was applied onto a lower left arm (i.e., between left wrist and left elbow) of the same subject. The subjects evaluated whether a low-temperature burn had occurred and whether the exothermic structure peeled off during its use. They also evaluated whether they felt a tingle at the removal of the structures.

Three pairs were tested, namely, a pair of production examples 1 and 2, another pair of production examples 2 and 3, and the other pair of production examples 3 and 1. Tables 3 to 5 show the results.

**TABLE 3**

| Comparison between Production examples 1 and 2 | | | |
|---|---|---|---|
| | | Production example 1 | Production example 2 |
| Low-temperature burn | Burned | 0 | 0 |
| | Not burned | 10 | 10 |
| Peeled off during use | Peeled off | 0 | 1 |
| | Not peeled off | 10 | 9 |
| Tingle at removal | Tingled | 0 | 0 |
| | Tingled a little | 2 | 0 |
| | Not tingled | 8 | 10 |

**TABLE 4**

| Comparison between Production examples 2 and 3 | | | |
|---|---|---|---|
| | | Production example 2 | Production example 3 |
| Low-temperature burn | Burned | 0 | 0 |
| | Not burned | 10 | 10 |
| Peeled off during use | Peeled off | 1 | 1 |
| | Not peeled off | 9 | 9 |
| Tingle at removal | Tingled | 0 | 0 |
| | Tingled a little | 0 | 1 |
| | Not tingled | 10 | 9 |

**TABLE 5**

| Comparison between Production examples 3 and 1 | | | |
|---|---|---|---|
| | | Production example 3 | Production example 1 |
| Low-temperature burn | Burned | 0 | 0 |
| | Not burned | 10 | 10 |
| Peeled off during use | Peeled off | 1 | 0 |
| | Not peeled off | 9 | 10 |
| Tingle at removal | Tingled | 0 | 0 |
| | Tingled a little | 1 | 2 |
| | Not tingled | 9 | 8 |

The results shown in Tables 3 to 5 demonstrate that the low-temperature burn did not occur, there was little trouble that the exothermic structure peeled off during its use, and the degree of the tingle at the removal was low when the exothermic structures of this invention were used.

### EXAMPLE 2

### (Production of Exothermic Structure 4)

The exothermic structure (production example 4) was produced in the same manner as that of the production of the exothermic structure 2, except that the adhesive layer 37 that is directly applied to skin was entirely formed on one surface of the release sheet.

In the production example 4, the total area of adhesive layer 37/the total area of release sheet 39 was 100%, the total area of adhesive portions/the total area of adhesive layer 37 was about 80%, and the total length of adhesive portions/the total length of adhesive layer 37 in the outer periphery was about 67%.

### (Test Example)

By using the exothermic structures of production examples 2 and 4, the following tests were conducted.

### I. Determination of Temperature

By using a test apparatus comprising a warmer and a constant-temperature bath of a water-circulation type, the temperature of the surface of the air-impermeable side was determined according to the method of JIS S 4100(1996).

### (1) Warmer

The warmer is made of SUS 304 having a thickness of 3 mm that is determined by JIS G 4303 (Hot-rolled Stainless Steel Plate) and has a form of a box having sizes of 300 mm (length), 600 mm (width), and 100 mm (height). On the superior surface (300 mm x 600 mm) of the warmer, a polypropylene resin plate having a thickness of 6 mm is fixed with screws. The surfaces other than the superior surface are covered with foamed polystyrene insulations each having a thickness of 30 mm. Warm water can circulate inside the warmer.

### (2) Constant-temperature Bath of Water-circulation Type

It is a bath that can circulate warm water in a flow rate of 12 ± 2 liter per minute. In this test, the temperature of the warm water was controlled so that the temperature of the surface of the polypropylene resin plate comes to 35 to 37 degrees Celsius.

### (3) Test method

After the exothermic structure was taken out from the airproof outer bag, the release sheet was removed, and then the adhesive layer of the air-impermeable side was applied onto the surface of the polypropylene resin plate. A thermometer was inserted between the exothermic structure and the polypropylene resin plate, and the temperature was determined for eight hours. Table 6 shows the highest temperature in eight hours and the temperature after 8 hours.

### II. Determination of Adhesive Force

The adhesive force of the adhesive layer of the exothermic structure was determined by the method (Test Method for Adhesive Tape and Adhesive Sheet; Ball Tack Method with Slope) according to JIS Z 0237(2000). Table 6 shows the results.

**TABLE 6**

| | Highest temperature | Temperature after 8 hours | Adhesive force^{*} |
|---|---|---|---|
| Production example 2 | 45.8°C | 40.6°C | 14 |
| Production example 4 | 45.3°C | 40.4°C | 14 |

| | | | |
|---|---|---|---|
| Note : It is given by the ball number of the steel ball. No. 32 shows the highest adhesive force. | | | |

As is clear from Table 6, the exothermic structure of this invention (Production example 2) exhibited an exothermic property and an adhesive property that were the same as those of the prior exothermic structure (Production example 4).

### III. Use Test of Exothermic Structure

One group consisted of ten subjects. Specifically, there were two subjects that evaluated by themselves to have much body hair, three subjects that evaluated by themselves to have some body hair, and five subjects that evaluated by themselves to have body hair in a normal amount.

One exothermic structure was applied onto a lower right arm (i.e., between right wrist and right elbow) of a subject and another exothermic structure was applied onto a lower left arm (i.e., between left wrist and left elbow) of the same subject. The subjects evaluated whether a low-temperature burn had occurred, whether they felt irritation in skin, and whether the exothermic structure peeled off during its use, namely, in eight hours. Table 7 shows the results.

**TABLE 7**

| Comparison between Production examples 2 and 4 | | | |
|---|---|---|---|
| | | Production example 2 | Production example 4 |
| Low-tempearture burn | Burned | 0 | 1 |
| | Not burned | 10 | 9 |
| Irritation in skin | Irritated | 0 | 3 |
| | Not irritated | 10 | 7 |
| Peeled off in use | Peeled off | 0λ | 0λ |
| | Not peeled off | 10λ | 10λ |

The results shown in Table 7 demonstrate that the low-temperature burn or irritation in skin did not occur or that there was no trouble that the exothermic structure peeled off during its use when the exothermic structure (Production example 2) of this invention was used. In contrast, when the prior exothermic structure (Production example 4) was used, the low-temperature burn or irritation in skin occurred a little.

### EXAMPLE 3

### (Production of Exothermic Structure 5)

The exothermic structure 1,000 that was shown in Figure 1 and had an adhesive layer of Figure 8B was produced as follows:
(1) A porous film 1 made of a polyethylene (manufactured by KOUJIN CO., LTD.; TSF-EU; a thickness of 50 µm) was partly bonded to a spun lace non-woven fabric made of a polyester (manufactured by ASAHI KASEI CORPORATION; a basis weight of 60 g/m²). Thus, a sheet (the first sheet 10x) for an air-permeable side 10 was obtained. The moisture permeability (JIS K 7129(2008)) of the first sheet 10x was 310 g/m^{2.} 24hours by the Lyssy method.
(2) Separately, on one surface of a polyester film 39 having a silicone-coating layer (manufactured by TOYO METALLIZING CO., LTD., Cerapeel; a thickness of 38 µ m) that would be used as a release sheet, an adhesive of a styrene-isoprene-styrene block-copolymer type (manufactured by NIPPON NSC LTD.; ME126) was applied so that an adhesive layer that would be directly applied to skin for one exothermic structure would have the shape as shown in Figure 8B. Then, the adhesive was dried to form the adhesive layer 37 including portions 37u and 37v that would be directly applied to skin.
(3) The surface of the adhesive layer 37 including portions 37u and 37v that had formed in Step (2) was covered with a commercially available, air-impermeable polyethylene film 31 (manufactured by FUKUI MINASELL Co., LTD.; a thickness of 40 µm) having a size that was the same as that of the polyester film 39. Then, the adhesive layer 37 was transferred onto the air-impermeable polyethylene film 31.
(4) An acrylic, solvent-type bonding agent 32 (manufactured by NIPPON SYNTHETIC CHEMICAL INDUSTRY CO., LTD.) was applied onto the entire surface of the air-impermeable polyethylene film 31. Thus, a sheet for an air-impermeable side was formed. Figure 9A shows the portion that corresponds to the air-impermeable side 30c of one exothermic structure.
(5) On the surface of the porous film made of a polyethylene in the first sheet 10x, the exothermic composition 50 having a formula of Table 1 in Example 1 was put in an amount of 20 g per one exothermic structure (see Figure 9B).
(6) The surface of the exothermic composition 50 was covered with the sheet for an air-impermeable side, and with the acrylic, solvent-type bonding agent 32, the porous film made of a polyethylene in the first sheet 10x was bonded to the air-impermeable polyethylene film 31 of the sheet for an air-impermeable side. In this step, the pouch portions where the exothermic composition 50 existed were pressed so that the exothermic composition 50 had a thickness of about 1.5 mm. Thus, as shown in Figure 9C one in which there were a series (one behind the other and side by side) of plural exothermic structures was produced.
(7) With a die, the portions that had been bonded with the bonding agent 32 were cut at the center of width of each portion to give plural exothermic structures 1,000. Each of the exothermic structures was put into an airproof outer bag in a sealed state. The exothermic structure 1,000 thus produced had a size of 9.5 cm x 13 cm. The width of the adhesive layer 37 was 10 mm. The distance w between portions 37u and 37v was 20 mm.

### (Test Example)

After the exothermic structure 1,000 was taken out from the airproof outer bag, the release sheet 39 was removed. In this step, a top of a nail was inserted into the portion where there was no adhesive layer 37, namely, the portion shown as the distance w in Figure 8B. Thus, the release sheet 39 was smoothly peeled off.

## Claims

1. An exothermic structure that is directly applied to skin comprising a pouch having an air-permeable side and an air-impermeable side, and a exothermic composition that is incorporated into the pouch in a sealed state, wherein the air-impermeable side comprises an inner polymer sheet layer, an intermediate adhesive layer that is used to apply to skin, and an outer release sheet, wherein the adhesive layer exists in part or entire of an outer periphery on an inner surface of the release sheet, the total area of the adhesive layer is from 10 to 40% of the area of the release sheet, the adhesive layer has an adhesive portion(s) and a non-adhesive portion(s) or only an adhesive portion(s), the total area of the adhesive portion(s) is from 30 to 100% of the total area of the adhesive layer, and in the outer periphery the total length of the adhesive portion(s) is from 60 to 100% of the total length of the adhesive layer.

2. The exothermic structure according to claim 1, wherein a layer of a tackiness agent exists on an outer surface of the polymer sheet layer of the air-impermeable side, a fibrous layer is bonded onto the layer of the tackiness agent, and the adhesive layer exists between the fibrous layer and the release sheet.

3. The exothermic structure according to claim 1, wherein the adhesive layer exists in part of the outer periphery on the inner surface of the release sheet.

4. The exothermic structure according to claim 1, wherein on the inner surface of the release sheet the adhesive layer exists only in part or entire of a portion that corresponds to a portion where the air-permeable side and the air-impermeable side are bonded together.

5. The exothermic structure according to claim 4, wherein on the inner surface of the release sheet the adhesive layer exists only in part of the portion that corresponds to the portion where the air-permeable side and the air-impermeable side are bonded together.

6. The exothermic structure according to claim 5, wherein an inner periphery of the adhesive layer exists at a position that is outside of an inner periphery of the portion that corresponds to the portion where the air-permeable side and the air-impermeable side are bonded together.

7. The exothermic structure according to claim 1, wherein the distance between the outer periphery and the inner periphery of the adhesive layer is from 5 to 30 mm.

8. The exothermic structure according to claim 1, wherein the adhesive layer has the non-adhesive portion(s).

9. The exothermic structure according to claim 8, wherein the shape of the non-adhesive portion(s) is diamond.

10. The exothermic structure according to claim 3, wherein the adhesive layer has two U-like-shaped portions that are axisymmetric to each other, and the distance between the U-like-shaped portions is from 10 to 30 mm.

11. The exothermic structure according to claim 1, which has at least two pouches into which the exothermic composition is incorporated in a sealed state, and the pouches are connected to each other.

12. A method for preparing the exothermic structure according to claim 2, comprises (1) setting a first sheet that is to be an air-permeable side comprising an outer-most layer a that is at least one of the outer-most layers of the first sheet and a second sheet comprising a polymer sheet b and a layer of a tackiness agent that exists on the entire of one surface of the polymer sheet b and of which surface is protected by a release paper so that the outer-most layer a of the first sheet faces the polymer sheet b of the second sheet, wherein the outer-most layer a is a heat-sealable by itself or can be heat-sealed by the polymer sheet b of the second sheet, and the polymer sheet b is a heat-sealable by itself or can be heat-sealed by the outer-most layer a of the first sheet, (2) heat-sealing in a transverse direction (with the proviso that if a heat-sealed portion in the transverse direction has been previously made, this step is not conducted), (3) heat-sealing in a longitudinal direction to form parallel at least two pouch portions each having heat-sealed three sides, (4) putting an exothermic composition into those pouch portions, (5) heat-sealing in a transverse direction to make pouch portions into which the exothermic composition is put in a sealed state, (6) removing the release paper, (7) applying a sheet for a fibrous layer to the layer of the tackiness agent that has been bared, wherein the sheet comprises the fibrous layer, on portions that correspond to the outer peripheries of pouches that would be made and on one surface of the fibrous layer, an adhesive layer that is directly applied to skin, and a release sheet that covers the adhesive layer, so that a side of the fibrous layer on which side there is no adhesive layer faces the layer of the tackiness agent, (8) cutting, at need, the heat-sealed portion(s) of the longitudinal direction at a center of the width of the longitudinal heat-sealed portion(s), (9) simultaneously or posteriorly cutting the transverse heat-sealed portion at a center of the width of the transverse heat-sealed portion to give an exothermic structure(s), and then (10) putting the exothermic structure(s) into an air-proof outer bag(s) in a sealed state.

13. The method according to claim 12, wherein the cut at the center of the width of the longitudinal heat-sealed portion is done with a rotary blade and the cut at the center of the width of the transverse heat-sealed portion is done with a roll cutter.
